# EUROPÄISCHE PATENTSCHRIFT

(11) **EP 1 237 910 B1**
(45) Veröffentlichungstag und Bekanntmachung des Hinweises auf die Patenterteilung: **10.09.2008**
(21) Anmeldenummer: 00987076.7
(22) Anmeldetag: 06.11.2000
(51) Int. Cl.: C07K 14/00

(54) **MIT TRP-PROTEINEN VERWANDTES PROTEIN MTR1 UND DIESES CODIERENDE DNA-SEQUENZ**
TRP-PROTEIN-RELATED MTR1 PROTEIN AND DNA SEQUENCE CODING THEREFOR
PROTEINE MTR1 APPARENTEE AUX PROTEINES TRP ET SEQUENCE ADN CODANT POUR CELLE-CI

(30) Priorität: 04.11.1999 DE 19953167
(43) Veröffentlichungstag der Anmeldung: 11.09.2002
(73) Patentinhaber: Johannes Gutenberg-Universität Mainz, 55122 Mainz (DE)
(72) Erfinder: PRAWITT, Dirk, 55131 Mainz (DE); PELLETIER, Jerry, McIntyre Medical Sciences Blg., Montreal, Quebec H3G 1Y6 (CA); ZABEL, Bernhard, 55101 Mainz (DE)
(74) Vertreter: Schüssler, Andrea
(86) Internationale Anmeldenummer: PCT/DE2000/003876
(87) Internationale Veröffentlichungsnummer: WO 2001/032693

(56) Entgegenhaltungen:
- WO-A-00/40614
- WO-A-99/35158
- DATABASE EMBL [Online] Accession No. AC003693, 19. Dezember 1997 (1997-12-19) EVANS, GA ET AL: "Human Chromosome 11p15.5 PAC clone pDJ915f1 containing KvLQT1 gene, complete sequence" XP002167907
- PRAWITT D ET AL: "Cloning and characterization of genes from Wilms' tumor candidate regions." AMERICAN JOURNAL OF HUMAN GENETICS, Bd. 65, Nr. 4, Oktober 1999 (1999-10), Seite A316 XP000999718 49th Annual Meeting of the American Society of Human Genetics;San Francisco, California, USA; October 19-23, 1999 ISSN: 0002-9297
- NAKAMORI R ET AL: "MTR1;a novel protein which related to the ooplasmic segregation of the ascidian Ciona intestinalis." ZOOLOGICAL SCIENCE (TOKYO), Bd. 15, Nr. SUPPL., Dezember 1998 (1998-12), Seite 62 XP000999008 Sixty-Ninth Annual Meeting of the Zoological Society of Japan;Hiroshima, Japan; September 26-28, 1998 ISSN: 0289-0003
- PRAWITT DIRK ET AL: "Identification and characterization of MTR1, a novel gene with homology to melastatin (MLSN1) and the trp gene family located in the BWS-WT2 critical region on chromosome 11p15.5 and showing allele-specific expression." HUMAN MOLECULAR GENETICS, Bd. 9, Nr. 2, 22. Januar 2000 (2000-01-22), Seiten 203-216, XP000996495 ISSN: 0964-6906

## Beschreibung

Die vorliegende Erfindung betrifft das Protein MTR1, das zur TRP-Familie gehört und, an der Ca²⁺- Regulation innerhalb der Zelle beteiligt ist, und/oder mit der Ätiologie von BWS und/oder mit 11p15.5-Veränderungen assoziierten Tumoren in Zusammenhang steht.

Anhand der Ergebnisse von zytogenetischen und molekularen Studien wurden verschiedene Erkrankungen mit dem humanen chromosomalen Bereich 11p15.5 in Verbindung gebracht. Die vorherrschende und die höchste Komplexität aufweisende Erkrankung ist dabei das Beckwith-Wiedemann Syndrom (BWS), das in einer Häufigkeit bei ca. einer von 13700 Geburten auftritt. Die Hauptmerkmale dieser ererbten Erkrankung sind Nabelbruch, Makroglossie und Riesenwuchs kombiniert mit einem erhöhten Risiko für die Entwicklung von Tumoren, z.B. Wilms Tumor (WT), Rhabdoid-Tumore und Rhabdomyosarcoma. Obwohl die Mehrzahl der BWS-Fälle nicht familär gehäuft (= "sporadisch") auftritt, segregieren einige wenige Fälle autosomal dominant mit einem praktisch ausschließlich mütterlichen Vererbungsgang. In mit BWS in Zusammenhang stehenden Tumoren wird häufig der Verlust der Heterozygozität (LOH) für 11p15.5 unter Beteiligung des mütterlichen Allels beobachtet. Die beobachtete funktionelle Ungleichheit der väterlichen und mütterlichen Allele in somatischen Zellen beruht auf einer epigenetischen Modifikation, die "genomic imprinting" genannt wird. Es konnte gezeigt werden, daß die Störung dieses "imprinting" zu einer Entwicklungs-Fehlregulation führt, die wiederum in Fehlbildungen und bösartigen Tumoren resultiert. Der Verlust des "imprinting" (LOI) von 11p15.5-Genen findet sich häufig in mit BWS in Zusammenhang stehenden Tumoren. Inzwischen wurde ein Bereich zwischen den chromosomalen Markern D11S648 und D11S1318 des Chromosoms 11 kartiert. Dieser umfaßt die "BWS critical region 1" (BWSCR1: D11S679-D11S551), welche einen Bereich darstellt, der durch die Bruchstellen chromsomaler Rearrangements in BWS-Patienten definiert wurde.

Verschiedene 11p15-Gene mit Funktionen, die mit dem Wachstum in Zusammenhang stehen, wurden bereits charakterisiert. Dazu gehört beispielsweise IGF2. IGF2 ist ein väterlich transkribierter mit Insulin verwandter Faktor für die Wachstumsregulation mit einer Schlüsselrolle bei der Hormon-gesteuerten Zellproliferation. Eine signifikante Anzahl von BWS-Patienten bzw. Patienten mit Wilms Tumor zeigen hinsichtlich IGF2 eine einen Elternteil betreffende Disomie (patUPD; patUPD = paternale uniparentale Disomie, d.h. das väterliche Allel [hier: von IGF2] liegt doppelt vor, das mütterliche fehlt) oder einen Verlust der Heterozygozität (LOH).

Database EMBL, Accession No. AC 0003693 zeigt eine ca. 155000 Nukleotide lange genomische Sequenz des 11p15.5. Chromosoms.

WO-A-99/35158 beschreibt 36 unterschiedliche humane sekretierte Proteine und die diese kodierenden genetischen Sequenzen.

WO-A-00/40614 beschreibt die Charakterisierung von Nukleinsäuren und Proteinen des Calciumkanals.

Somit liegt der vorliegenden Erfindung das technische Problem zugrunde, Gene bzw. deren Produkte zu identifizieren und bereitzustellen, die mit BWS und/oder mit BWS assoziierten Tumoren in Zusammenhang stehen und gegebenenfalls von diagnostischem und/oder therapeutischem Nutzen sind.

Die Lösung dieses technischen Problems wird durch die Bereitstellung der in den Patentansprüchen gekennzeichneten Ausführungsformen erzielt.

Es wurde überraschenderweise ein Gen (MTR1) innerhalb von menschlichen Chromosom 11 (11p15.5) gefunden, das über die vorstehenden Eigenschaften verfügt.

MTR1 ist in 11p15.5 zwischen TSSC4 (= Gen Nr. 4 aus einem 2,5 Mb "tumor-suppressing subchromosomal transferable fragment" der humanen Chromosomenregion 11p15) und KvLQT1 (auf Chromosom 11 lokalisiertes LQT1-Gen) lokalisiert und wird als 4,5 kb Transkript in verschiedenen fötalen Geweben und Geweben von Erwachsenen transkribiert. TSSG4 und KvLQT1 sind bekannte Gene, die in 11p15.5 lokalisiert sind und hier als chromosomale Marker dienen. Der offene Leserahmen von MTR1 teilt sich in 24 Exons auf, von denen Exon 18 alternativ gespleißt wird, was zu zwei Proteinen mit 872 bzw. 1165 Aminosäuren führt . Die Menge der MTR1-Transkripte ist in Wilms Tumoren und Rhabodmyosarcomen erhöht. MTR1 kartiert in der Nähe einer Translokations bruchstelle in der rhabdoiden Tumorzellinie TM87-16. Darüber hinaus wird im GM-Hybridzellsystem MTR1 nur vom väterlichen Chromosom 11 transkribiert, was auf eine allelspezifische Inaktivierung der mütterlichen Kopie durch "genetic imprinting" hinweist.

Das von MTR1 codierte Protein gehört zur Trp (transient receptor potential)-Proteinfamilie. Diese Zugehörigkeit ergibt sich nicht allein aus Sequenzhomologien, sondern auch die Transmembran-Domänen in MTR1 sind in einer Anzahl geclustert und weisen Zwischenräume auf, die denen der Transmembran-Domänen der TRP-Genfamilie entsprechen. Ein hochkonserviertes Motiv (EWKFAR) kurz nach der letzten Transmembran-Domäne ist in den Proteinen vorhanden, die von mehr als 90% aller TRP-Gene codiert werden. Die TRP-Genfamilie ist für den Agonisten-aktivierten Ca²⁺-Eintritt in Zellen ausschlaggebend. Durch den Einstrom der Ionen wird der Ca²⁺-Vorrat, der durch eine Reihe von Stimuli aufgebraucht wird, aufgefrischt und dieser ist auch essentiell für angemessene zelluläre Antworten gegenüber Hormonen und/oder Wachstumsfaktoren. Für IGF-1, das wie IGF-2 ein Wachstumsfaktor ist, konnte gezeigt werden, daß dieses das Fortschreiten des Zellzyklus in verschiedenen Zelltypen fördert und auch bei der Tumorbildung eine wichtige Rolle spielt. Da die Hemmung des Calciums-Eintritts die Wachstum-fördernden Wirkungen von IGF-1 hemmt, ist die Stimulation des Calcium-Eintritts für die Wachstumsinduktion essentiell. Der Bereich der höchsten Homologie zwischen MTR1 und den Trp-Genen ist um den Porenbereich und der letzten Transmembran-Domäne zu finden, was die Funktion von MTR1 bei der Ca²⁺-Regulation in Zellen nahelegt.

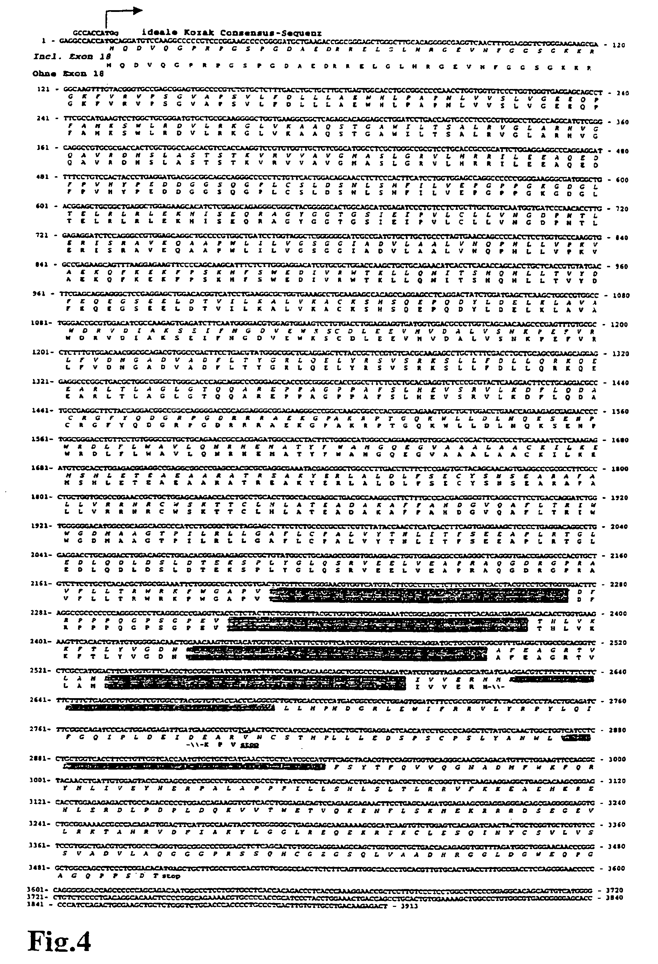

Gegenstand der vorliegenden Erfindung ist eine cDNA-Sequenz, die ein Protein (MTR1) mit einer der in Figur 4 gezeigten Aminosäuresequenzen codiert, wobei das Protein (MTR1) zumindest eine biologische Aktivität eines Proteins der TRP-Familie aufweist/oder und an der Ätiologie von BWS und/oder von mit 11p15.5-Veränderungen assoziierten Tumoren in Zusammenhang steht. Vorzugsweise umfaßt diese cDNA-Sequenz eine der in Figur 4 gezeigten DNA-Sequenzen.

In einer bevorzugten Ausführungsform betrifft die vorliegende Erfindung eine cDNA-Sequenz, die für ein Protein (MTR1) der TRP-Familie codiert, wobei das Protein an der Ca²⁺-Regulation innerhalb der Zelle beteiligt ist.

Die erfindungsgemäßen cDNA-Sequenzen sind für verschiedene Zwecke von Nutzen. So können sie, wie nachstehend näher beschrieben, zur rekombinanten Herstellung des MTR1-Proteins für Analysezwecke, zur weiteren Charakterisierung oder für therapeutische Zwecke verwendet werden. Sie können auch als Marker für Gewebe dienen, in denen das entsprechende Protein bevorzugt exprimiert wird (entweder konstitutiv oder während eines bestimmten Stadiums hinsichtlich der Gewebedifferenzierung, Entwicklung eines Krankheitsstadiums etc.). Darüber hinaus können sie auch als chromosomale Marker oder als "tages" zur Identifizierung von Chromosomen dienen bzw. zur Kartierung von verwandten Genen. Ferner können die erfindungsgemäßen DNA-Sequenzen auch zum Vergleich mit endogenen DNA-Sequenzen von Patienten verwendet werden, um so potentielle genetische Erkrankungen identifizieren zu können. Sie können darüber hinaus als Hybridisierungs-Sonden zur Identifizierung neuer, verwandter DNA-Sequenzen dienen oder als Informationsquelle zur Entwicklung von PCR-Primern, beispielsweise für genetisches "fingerprinting". Schließlich können sie zur Auswahl und Herstellung von Oligonukleotiden zur Beschichtung eines "Genchips" oder eines anderen Trägers dienen, beispielsweise in Bezug auf das Studium von Expressionsmustern, zur Erzeugung von anti-Protein-Antikörpern, beispielsweise mittels DNA-Immunisierungsverfahren, und als Antigen zur Erzeugung von anti-DNA-Antikörpern oder zur Auslösung einer weiteren Immunantwort.

Durch die Erniedrigung oder Hemmung der Expression der vorstehend beschriebenen DNA-Sequenzen, kann die Synthese von MTR1 bzw. verwandten Proteinen verringert oder eliminiert werden, was beispielsweise bei den vorstehend beschriebenen Krankheitszuständen wünschenswert sein kann. Daher betrifft eine weitere bevorzugte Ausführungsform der vorliegenden Erfindung eine Antisense-RNA, die dadurch gekennzeichnet ist, daß sie zu den vorstehenden cDNA-Sequenzen komplementär ist und an die davon translatierte RNA spezifisch binden kann und die Synthese des von diesen DNA-Sequenzen codierten MTR1-Proteins oder des damit verwandten Proteins verringern oder hemmen kann, und ein Ribozym, das dadurch gekennzeichnet ist, daß es zu den vorstehenden cDNA-Sequenzen komplementär ist und an die von diesen cDNA-Sequenzen transkribierte RNA spezifisch binden und diese spalten kann, wodurch die Synthese des von diesen cDNA-Sequenzen codierten MTR1-Proteins ebenfalls verringert oder gehemmt wird. Vorzugsweise sind diese Antisense-RNAs und Ribozyme zu einer codierenden Region der mRNA komplementär. Der Fachmann ist in der Lage, ausgehend von den offenbarten cDNA-Sequenzen, geeignete Antisense-RNAs herzustellen und anzuwenden. Geeignete Vorgehensweisen sind beispielsweise in EB-B1 0 223 399 oder EP-A1 0 458 beschrieben. Ribozyme sind RNA-Enzyme und bestehen aus einem einzelnen RNA-Strang. Diese können andere RNAs intermolekular spalten, beispielsweise die von den erfindungsgemäßen DNA-Sequenzen transkribierten mRNAs. Diese Ribozyme müssen prinzipiell über zwei Domänen verfügen, (1) eine katalytische Domäne und, (2) eine Domäne, die zu der Ziel-RNA komplementär ist und an diese binden kann, was die Voraussetzung für eine Spaltung der Ziel-RNA ist. Ausgehend von in der Literatur beschriebenen Vorgehensweisen ist es inzwischen möglich, spezifische Ribozyme zu konstruieren, die eine gewünschte RNA an einer bestimmten, vorgewählten Stelle schneiden (siehe beispielsweise Tanner et al., in: Antisense Research and Applications, CRC Press, Inc. (1993), 415-426). Vorzugsweise hybridisieren die vorstehenden Ribozyme oder Antisense-RNAs über einen Bereich von mindestens 15, mehr bevorzugt mindestens 25 und am meisten bevorzugt mindenstens 50 Blasen mit der von den erfindungsgemäßen cDNA-Sequenzen transkribierten RNA.

Die erfindungsgemäßen DNA-Sequenzen bzw. die die vorstehend beschriebenen Aritisense-RNAs oder Ribozyme codierenden cDNAs können auch in einen Vektor bzw. Expressionsvektor inseriert werden. Somit umfaßt die vorliegende Erfindung auch diese DNA-Sequenzen enthaltende Vektoren bzw. Expressionsvektoren. Die Bezeichnung "Vektor" bezieht sich auf ein Plasmid (pUC18, pBR322, pBlueScript etc.), auf ein Virus oder ein anderes geeignetes Vehikel. In einer bevorzugten Ausführungsform sind die erfindungsgemäßen cDNA-Sequenzen im Vektor mit regulatorischen Elementen funktionell verknüpft, die deren Expression in prokaryotischen oder eukaryotischen Wirtszellen erlauben. Solche Vektoren enthalten neben den regulatorischen Elementen, beispielsweise einem Promotor, typischerweise einen Replikationsursprung und spezifische Gene, die die phänotypische Selektion einer transformierten Wirtszelle erlauben. Zu den regulatorischen Elementen für die Expression in Prokaryoten, beispielsweise E.coli, zählen der lac-, trp-Promotor, T3- oder T7-Promotor, und für die Expression in Eukaryoten der AOX1- oder GAL1-Promotor in Hefe und der CMV-, SV40-, RVS-40-Promotor, CMV-oder SV40-Enhancer für die Expression in tierischen Zellen. Weitere Beispiele für geeignete Promotoren sind der Metallothionein I- und der Polyhedrin-Promotor. Zu geeigneten Expressionsvektoren für E.coli zählen beispielsweise pGEMEX, pUC-Derivate, pGEX-2T, pET3b und pQE-8, wobei letzterer bevorzugt ist. Zu den für die Expression in Hefe geeigneten Vektoren zählen pY100 und Ycpad1, für die Expression in Säugerzellen pMSXND, pKCR, pEFBOS, cDM8 und pCEV4. Zu den erfindungsgemäßen Expressionsvektoren zählen auch von Baculovirus abgeleitete Vektoren für die Expression in Insektenzellen, beispielsweise pAcSGHisNT-A.

Allgemeine, auf dem Fachgebiet. bekannte Verfahren können zur Konstruktion von Expressionsvektoren, die die erfindungsgemäßen cDNA-Sequenzen und geeignete Kontrollsequenzen enthalten, verwendet werden. Zu diesen Verfahren zählen beispielsweise in vitro-Rekombinationstechniken, synthetische Verfahren, sowie in vivo-Rekombinationsverfahren, wie sie beispielsweise in Sambrook et al., supra, beschrieben sind. Die erfindungsgemäßen cDNA-Sequenzen können auch in Verbindung mit einer für ein anderes Protein bzw. Peptid codierenden DNA inseriert werden, so daß die erfindungsgemäßen DNA-Sequenzen beispielsweise in Form eines Fusionsproteins exprimiert werden können.

Die vorliegende Erfindung betrifft auch die vorstehend beschriebenen Vektoren enthaltende Wirtszellen. Zu diesen Wirtszellen zählen Bakterien (beispielsweise die E.coli-Stämme HB101, DH1, x1776, JM101, JM109, BL21 und SG13009), Hefe, vorzugsweise S. cerevisiae, Insektenzellen, vorzugsweise sf9-Zellen, und Tierzellen, vorzugsweise Säugerzellen. Bevorzugte Säugerzellen sind CHO-, VERO-, BHK-, HeLa-, COS-, MDCK, 293- und WI38-Zellen. Verfahren zur Transformation dieser Wirtszellen, zur phänotypischen Selektion von Transformanten und zur Expression der erfindungsgemäßen cDNA-Moleküle unter Verwendung der vorstehend beschriebenen Vektoren sind auf dem Fachgebiet bekannt.

Die vorliegende Erfindung betrifft ferner ein von den erfindungsgemäßen cDNA-Sequenzen codiertes MTR1-Protein. Die vorliegende Erfindung betrifft weiter Verfahren zur rekombinanten Herstellung des MTR1-Proteins unter Verwendung der erfindungsgemäßen Expressionsvektoren: Das erfindungsgemäße Verfahren umfaßt die Kultivierung der vorstehend beschriebenen Wirtszellen unter Bedingungen, die die Expression des Proteins (bzw. Fusionsproteins) erlauben (vorzugsweise stabile Expression), und die Gewinnung des Proteins aus der Kultur oder aus den Wirtszellen. Dem Fachmann sind Bedingungen bekannt, transformierte bzw. transfizierte Wirtszellen zu kultivieren. Geeignete Reinigungsverfahren (beispielsweise präparative Chromatographie, Affinitätschromatographie, beispielsweise Immunoaffinitätschromatographie, HPLC etc.) sind ebenfalls allgemein bekannt. Das erfindungsgemäße MTR1-Protein bzw. das damit verwandte Protein ist nicht nur bei den nachstehend beschriebenen therapeutischen Maßnahmen von Nutzen, sondern kann beispielsweise auch in biologischen Assays hinsichtlich einer Aktivitätsbestimmung Verwendung finden, beispielsweise in Kombination mit einer Reihe einer Vielzahl von weiteren Proteinen für ein "high-throughput"-Screenen. Darüber hinaus kann es zur Erzeugung von Antikörpern oder zur Erzeugung einer anderen Immunantwort nützlich sein, als Reagenz (beispielsweise in markierter Form) in kompetitiven Assays zur quantitativen Bestimmung des Proteins in biologischen Flüssigkeiten, als Marker für Gewebe, in denen das entsprechende Protein bevorzugt erzeugt wird (entweder konstitutiv oder während eines bestimmten Stadiums hinsichtlich der Gewebedifferenzierung, Entwicklung eines Krankheitsstadiums etc.), und natürlich zur Isolierung von interagierenden Proteinen, d.h. beispielsweise Proteinen, die an der Ca²⁺-Ionehkanalbildung beteiligt sind. Schließlich kann das erfindungsgemäße MTR1-Protein bzw. das damit verwandte Protein auch zur Isolierung von Inhibitoren, Antagonisten oder Agonisten (beispielsweise hinsichtlich der Zusammenlagerung für die Ca²⁺-Ionenkanalbildung) verwendet werden, wobei es sich beispielsweise um Peptide oder sonstige kleine Moleküle handeln kann.

Eine weitere bevorzugte Ausführungsform der vorliegenden Erfindung betrifft Liganden, beispielsweise Antikörper gegen das vorstehend beschriebene erfindungsgemäße MTR1-Protein. Diese Liganden können beispielsweise in diagnostischen Assays verwendet werden oder für therapeutische Zwecke, wobei es nach Anlagerung an das Zielmolekül, d.h. MTR1, zu dessen Hemmung kommt. Verfahren zur Isolierung bzw. Synthese solcher Liganden sind dem Fachmann bekannt. Beispielsweise können möglicherweise nützliche aktivitätshemmende Verbindungen in Extrakten von natürlichen Produkten als Ausgangsmaterial gescreent werden. Solche Extrakte können beispielsweise aus Pilzen, Actinomyceten, Algen, Insekten, Protozoen, Pflanzen und Bakterien stammen.

Vorzugsweise handelt es sich bei dem erfindungsgemäßen Liganden um Antikörper oder Fragmente davon, die an das MTR1-Protein spezifisch binden. Diese Antikörper können monoclonale, polyclonale oder synthetische Antikörper sein oder Fragmente davon. In diesem Zusammenhang bedeutet der Begriff "Fragment" alle Teile des monoclonalen Antikörpers (z.B. Fab-, Fv- oder "single chain Fv"-Fragmente), welche die gleiche Epitopspezifität wie der vollständige Antikörper aufweisen. Die Herstellung solcher Fragmente ist dem Fachmann bekannt. Vorzugsweise handelt es sich bei den erfindungsgemäßen Antikörpern um monoclonale Antikörper. Die erfindungsgemäßen Antikörper können gemäß Standardverfahren hergestellt werden, wobei vorzugsweise das von den erfindungsgemäßen cDNA-Sequenzen codierte MTR1-Protein oder ein synthetisches Fragment davon als Immunogen dienen. Verfahren zur Gewinnung monoclonaler Antikörper sind dem Fachmann bekannt.

In einer besonders bevorzugten Ausführungsform ist der genannte monoclonale Antikörper ein aus einem Tier (z.B. Maus) stammender Antikörper, ein humanisierter Antikörper oder ein chimärer Antikörper oder ein Fragment davon. Chimäre, menschlichen Antikörper ähnelnde oder humanisierte Antikörper besitzen eine herabgesetzte potentielle Antigenität, jedoch ist ihre Affinität gegenüber dem Ziel nicht herabgesetzt. Die Herstellung von chimären und humanisierten Antikörpern bzw. von den menschlichen Antikörpern ähnelnden Antikörpern wurde ausführlich beschrieben (siehe beispielsweise Queen et al., Proc. Natl. Acad. Sci. USA 86 (1989), 10029, und Verhoeyan et al., Science 239 (1988), 1534). Humanisierte Immunglobuline weisen variable Grundgerüstbereiche auf, die im wesentlichen von einem humanen Immunglobulin stammen (mit der Bezeichnung Akzeptor-Immunglobulin) und die Komplementarität der determinierenden Bereiche, die im wesentlichen von einem nicht-menschlichen Immunglobulin (z.B. von der Maus) stammen (mit der Bezeichnung Donor-Immunglobulin). Die (der) konstante(n) Bereich(e) stammt (stammen), falls vorhanden, auch im wesentlichen von einem menschlichen Immunglobulin. Bei der Verabreichung an menschliche Patienten bieten humanisierte (sowie die menschlichen) Antikörper eine Reihe von Vorteilen gegenüber Antikörpern von Mäusen oder anderen Spezies: (a) das menschliche Immunsystem sollte das Grundgerüst oder den konstanten Bereich des humanisierten Antikörpers nicht als fremd erkennen und daher sollte die Antikörperantwort gegen einen solchen injizierten Antikörper geringer ausfallen als gegen einen vollständig fremden Maus-Antikörper oder einen partiell fremden chimären Antikörper; (b) da der Effektorbereich des humanisierten Antikörpers menschlich ist, interagiert er besser mit anderen Teilen des menschlichen Immunsystems, und (c) injizierte humanisierte Antikörper weisen eine Halbwertszeit auf, die im wesentlichen zu der von natürlich vorkommenden menschlichen Antikörpern äquivalent ist, was es erlaubt, kleinere und weniger häufige Dosen im Vergleich zu Antikörpern anderer Spezies zu verabreichen.

Die erfindungsgemäßen Antikörper können beispielsweise auch zur Immunpräzipitation des erfindungsgemäßen MTR1-Proteins, zur Isolierung verwandter Proteine aus cDNA-Expressionsbanken oder für diagnostische Zwecke (siehe unten) verwendet werden. Die vorliegende Erfindung betrifft auch ein Hybridom, das den vorstehend beschriebenen monoclonalen Antikörper erzeugt.

Die vorliegende Erfindung erlaubt auch die Durchführung therapeutischer Maßnahmen bei den vorstehend diskutierten Erkrankungen, d.h. die vorstehend beschriebenen, erfindungsgemäßen cDNA-Sequenzen, Antisense-RNAs, Ribozyme, Vektoren und Antikörper können auch zur Herstellung eines Arzneimittels, beispielsweise zur Kontrolle der Expression des MTR1-Proteins (oder des damit verwandten Proteins) oder zum Austausch einer mutierten Form des Gens gegen eine funktionelle Form verwendet werden, somit beispielsweise einerseits zur Herstellung eines Arzneimittels zur Prävention oder der Behandlung von den vorstehend beschriebenen Erkrankungen, die offensichtlich mit einer veränderten Expression von MTR1 assoziiert sind, andererseits zur Herstellung eines Arzneimittels zur Prävention oder der Behandlung von Erkrankungen, die beispielsweise mit einer fehlerhaften Regulation des Ca²⁺-Eintritts in die Zellen assoziiert sind. Dies kann einerseits entweder eine Hemmung der Expression des MTR1-Gens (über Antisense-RNAs oder Ribozyme) oder eine Hemmung der Aktivität des MTR1-Proteins (beispielsweise mittels Antikörpern) andererseits eine Steigerung der Expression oder der Aktivität des Proteins (durch Applikation des Proteins selbst oder eines das Protein exprimierenden Vektors) erforderlich machen. Beispielsweise kann dazu das erfindungsgemäße MTR1-Protein in Säuger, insbesondere den Menschen, durch übliche Maßnahmen eingebracht werden. Hierzu kann es günstig sein, das Protein an ein vom jeweiligen Körper nicht als fremd angesehenes Protein, z.B. Transferrin oder Rinderserumalbumin (BSA), zu koppeln. Auch kann eine erfindungsgemäße cDNA-Sequenz, Antisense-RNA oder Ribozym in Säuger, insbesondere den Menschen, eingebracht und exprimiert werden.

Somit betrifft die vorliegende Erfindung auch ein Arzneimittel, das die vorstehend beschriebenen cDNA-Sequenzen, Antisense-RNA, das Ribozym, den Expressionsvektor, das erfindungsgemäße Protein oder Antikörper enthält. Dieses Arzneimittel enthält gegebenenfalls zusätzlich einen pharmazeutisch verträglichen Träger. Geeignete Träger und die Formulierung derartiger Arzneimittel sind dem Fachmann bekannt. Zu geeigneten Trägern zählen beispielsweise Phosphat-gepufferte Kochsalzlösungen, Wasser, Emulsionen, beispielsweise Öl/Wasser-Emulsionen, Netzmittel, sterile Lösungen etc. Die Verabreichung der Arzneimittel kann oral oder parenteral erfolgen. Zu den Verfahren für die parenterale Verabreichung gehören die topische, intra-arterielle, intramuskuläre, subkutane, intramedulläre, intrathekale, intraventrikuläre, intravenöse, intraperitoneale oder intranasale Verabreichung. Die geeignete Dosierung wird von dem behandelnden Arzt bestimmt und hängt von verschiedenen Faktoren ab, beispielsweise von dem Alter, dem Geschlecht, dem Gewicht des Patienten, dem Stadium der Erkrankung, der Art der Verabreichung etc..

Vorzugsweise werden die vorstehend beschriebenen cDNA-Sequenzen in einen für die Gentherapie geeigneten Vektor inseriert, beispielsweise unter Kontrolle eines gewebespezifischen Promotors, und in die Zellen eingeschleust. In einer bevorzugten Ausführungsform ist der die vorstehend beschriebenen cDNA-Sequenzen enthaltende Vektor ein Virus, beispielsweise ein Retrovirus, Adenovirus, adenoassoziiertes Virus oder Vaccinia-Virus. Besonders bevorzugt sind Retroviren. Beispiele für geeignete Retroviren sind MoMuLV, HaMuSV, MuMTV, RSV oder GaLV. Für Zwecke der Gentherapie können die erfindungsgemäßen DNA-Sequenzen auch in Form von kolloidalen Dispersionen zu den Zielzellen transportiert werden. Dazu zählen beispielsweise Liposomen oder Lipoplexe (Mannino et al., Biotechniques 6 (1988), 682). Diese Gentherapie kann sowohl für die Erhöhung der MTR1-Expression (beispielsweise mit einem Vektor, der das Gen für aktives MTR1-Protein enthält), als auch für eine Verringerung der MTR1-Expression (beispielsweise mit einem Vektor, der ein Gen für ein Ribozym, eine Antisense-RNA oder eine inaktive Form von MTR1 ("knock-out") enthält) Anwendung finden.

Die vorliegende Erfindung ermöglicht es auch, Störungen der MTR1-Expression auf genetischer Ebene zu untersuchen. Mit einer erfindungsgemäßen cDNA-Sequenz bzw. davon abgeleiteten Sonden oder Primern kann in Säugern, insbesondere dem Menschen, festgestellt werden, ob sie ein verändertes MTR1-Gen enthalten, das zu einer mutierten Form des Proteins führt, die nicht länger biologisch aktiv ist, oder ob beispielsweise MTR1 zu gering oder zu stark exprimiert wird. Dazu kann der Fachmann übliche Verfahren, wie Reverse Transkription, PCR, RT-PCR, LCR, Hybridisierung und Sequenzierung durchführen. Auch die erfindungsgemäßen Antikörper oder Fragmente davon, eignen sich für die Diagnostik, d.h. beispielsweise zum Nachweis des Vorhandenseins und/oder der Konzentration des MTR1-Proteins, einer verkürzten oder verlängerten Form dieses Proteins etc., in einer Probe. Die Antikörper können beispielsweise in Immunoassays in Flüssigphase oder an einen festen Träger gebunden werden. Dabei können die Antikörper auf verschiedene Art und Weise markiert sein. Geeignete Marker und Markierungsverfahren sind auf dem Fachgebiet bekannt. Beispiele für Immunassays sind ELISA und RIA. Eine MTR1-Sonde kann auch zur Diagnostik von patUPD oder LOH geeignet sein, z.B. wenn a) ein geeigneter Polymorphismus auf DNA-Ebene vorhanden ist, der den parentalen Ursprung einer MTR1-Kopie klären kann oder b) eine monoallelische Expression generell vorliegt, womit dann auf RNA-Ebene ein LOH oder LOI nachweisbar ist.

Somit betrifft die vorliegende Erfindung auch ein Diagnoseverfahren zum Nachweis einer gestörten MTR1-Expression, bei dem man eine Probe mit der erfindungsgemäßen cDNA-Sequenz oder dem Antikörper oder einem Fragment davon in Berührung bringt und sodann direkt oder indirekt bestimmt, ob sich die Konzentration, Länge und/oder Sequenz des MTR1-Proteins oder der dieses codierenden mRNA im Vergleich zu einer Kontrollprobe unterscheiden. Die für dieses Diagnoseverfahren verwendbaren Sonden umfassen auch auf den erfindungsgemäßen cDNA-Sequenzen basierende Primer, beispielsweise für eine PCR.

Schließlich betrifft die vorliegende Erfindung einen diagnostischen Kit zur Durchführung des vorstehend beschriebenen Diagnoseverfahrens, der eine erfindungsgemäße cDNA-Sequenz oder den vorstehend beschriebenen Antikörper oder das Fragment davon, enthält. Je nach Ausgestaltung des diagnostischen Kits können die cDNA-Sequenz bzw. der Antikörper immobilisiert sein.

Von den Erfindern wurde ferner herausgefunden, daß eine Verbindung von MTR1 zur Apoptose besteht sowie eine Beteiligung bei Zellwachstum, -tod und -differenzierung. Insbesondere spielt MTR1 wohl eine Rolle bei Erkrankungen des Urogenitaltrakts einschließlich polycystischer Nierenerkrankung (APKD).

Von den Erfindern wurde außer dem humanen Gen für MTR1 nun auch das Mausortholog isoliert. Die chromosomale Lokalisation ist auf dem distalen Chromosom 7. Dort wird es von den als chromosomalen Markern dienenden Genen TapaI und KvLQT1 flankiert. Das Maus-MTR1-Gen codiert ein 4,4 kB Transkript, das in einer Vielzahl von fötalen und adulten Geweben exprimiert wird. Der putative offene Leserahmen besteht aus 24 Exons, die 1158 Aminosäuren kodierten. Transmembran-Vorhersagealgorithmen zeigen die Anwesenheit von sechs Membran umspannenden Domänen an. Das Maus-MTR1-Gen zeigt eine biallelische Expression in allen getesteten Geweben verschiedender Entwicklungsstufen.

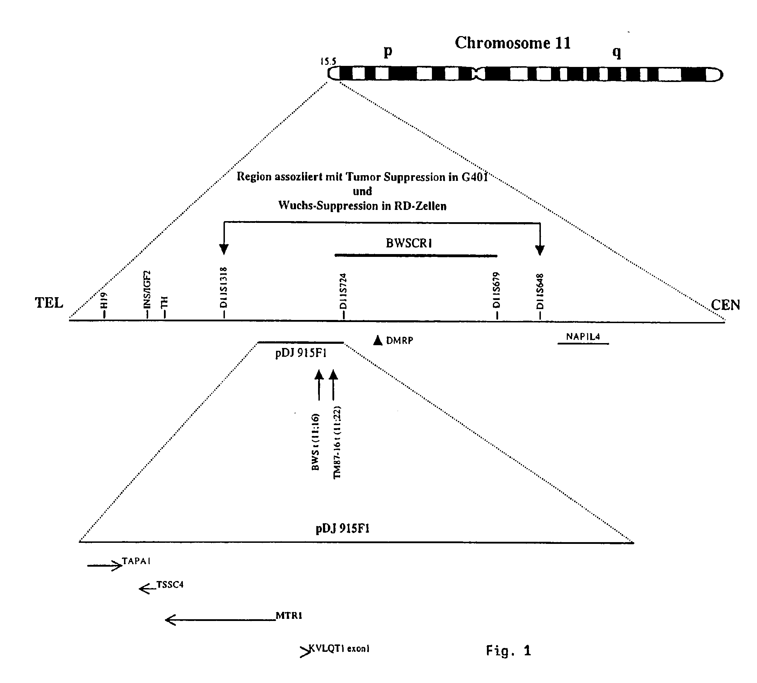

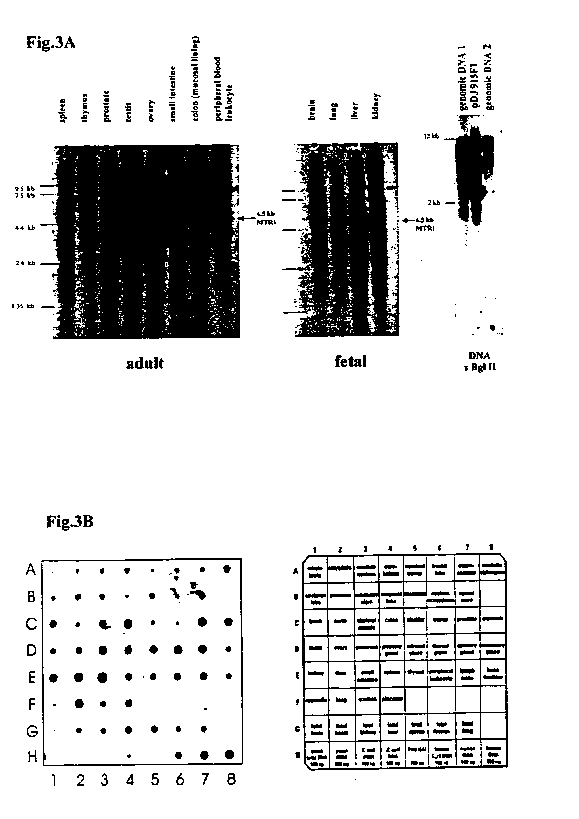

Kurze Beschreibung der Figuren:
Figur 1: Lokalisation von MTR1
   Analysiert wurde der PAC-Klon pDJ915F1, der humanes MTR1 enthält. Die Position des neuen Transkripts MTR1 in Relation zu weiteren Genen des Chromosoms 11 (Pfeile geben die Transkriptionsrichtung an) sind angegeben.
Figur 2: Genaue Exon/Intron-Übergänge des humanen MTR1-Gens
   Alle Exons (außer Exon 23A) korrelieren genau mit der **AG**-Exon-**GT**-Spleißregel für die Spleiß-Akzeptor/Speiß-Donor-Stellen (Exon 23A: **AG/GC**). Das humane MTR1-Transkript besteht aus 24 Exons, wobei Teile des 5' und 3' nichttranslatierten Bereichs fehlen. Die durchschnittliche Größe der Exons beträgt 151 bp. Exon 19 konnte bisher nicht auf RNA-Ebene verifiziert werden.
Figur 3
   3A: Northern-Blot-Ergebnisse hinsichtlich der humanen MTR1-RNA in fötalen Geweben und Geweben von Erwachsenen
   Die RNA-Quellen sind über den Probenspuren angegeben. Die ungefähren Fragmentpositionen der Molekulargewichtsmarker sind ebenfalls angegeben. Der Pfeil zeigt das Hybridisierungssignal für MTR1 bei 4,5 kb. Starke Signale wurden in folgenden Geweben von Erwachsenen erhalten: Prostata, Hoden, Eierstock, Colon und Leukozyten des peripheren Bluts. Starke Signale wurden auch in fötalem Gehirn, Leber und Niere erhalten. In den meisten anderen Spuren wurden schwache Banden beobachtet. Zum Ausschluß von Kreuzhybridisierung mit verwandten Genen wurde eine Southern-Blot mit BglII-geschnittenen genomischen DNAs und BglIIgeschnittenem genomischem pDJ915F1 unter identischen Bedingungen hybridisiert. In allen drei Spuren (rechts) wurden die gleichen Signale erhalten, somit handelt es sich um eine spezifische Hybridisierung ohne Anzeichen einer Kreuzhybridisierung.
   3B: Ergebnisse der Hybridisierung der Northern-Blots mit RNAs aus verschiedenen Geweben
   Es wurde die gleiche MTR1-Sonde wie in Figur 3A verwendet. Starke Hybridisierungssignale wurden sowohl in Niere, Leber, Lunge, Dünndarm, Pankreas und Drüsengewebe von Erwachsenen als auch in fötaler Niere, Leber, Milz und Thymus beobachtet.
Figur 4: Humane MTR1-cDNAs und ORF
   Es sind zwei alternative Spleißvarianten dargestellt. Die Spleißvariante 1 (kursiv) enthält Exon 18 und besitzt einen codierenden Bereich von 1165 Aminosäuren mit sechs Transmembran-Domänen (in Fettdruck), während bei Alternative 2 Exon 18 fehlt. Dies führt zu einem vorzeitigen Stop nach den ersten 872 Aminosäuren, wobei nur die ersten vier Transmembran-Domänen umfaßt werden. Die Position von Exon 18 wird von "-//-" Zeichen flankiert. Eine ideale "Kozak consensus"-Sequenz ist oben am Beginn des ORF angegeben. Lediglich das Nucleotid an der letzten Position von MTR1 unterscheidet sich von der idealen "Kozak"-Sequenz (ATGC anstelle von ATGG), was jedoch trotzdem zu einer funktionellen Initiation der Translation führt.
Figur 5
   5A: Hybridisierungssignale auf einem Southern-Blot mit DNAs der Zellinie GM
   GM-Zellinien enthalten nur den mütterlichen (7300, 13400) oder väterlichen (11941, 11944, 1927B) Bereich von 11p15.5 (in einem Nagerhintergrund). Mütterliche Hybridisierungssignale sind bei 2,4 kb, väterliche bei 2,7 kb, was die Beibehaltung des Epigenesis-Chromatinstadiums in den fünf GM-Zellinien zeigt.
5B: RT-PCR-Produkte aus GM-Zellinien
   Zur Verhinderung der Amplifikation auf der genomischen Ebene wurden RT-Reaktionen, wie in Beispiel 1 beschrieben, durchgeführt. Zur Verifizierung einer spezifischen Amplifikation wurden die Produkte sequenziert. Der obere Abschnitt zeigt RT-PCR-Produkte eines Teils der codierenden Region von NAP1L4 (siehe auch Figur 1) in allen fünf somatischen Zellhybriden, was die Beibehaltung von 11p15.5 und die Transkription von biallelisch transkribierten Genen darin zeigt (Kontrolle). Der untere Abschnitt zeigt die Amplifikation von MTR1-Produkten bei zwei der drei väterlichen Hybride (GM11927B, GM11944). Die Hybridisierung des geblottenen Gels mit einer MTR1-Sonde ergab auch ein schwaches Signal in GM11941 (nicht gezeigt), was eine lediglich väterliche Expression in MTR1 anzeigt.

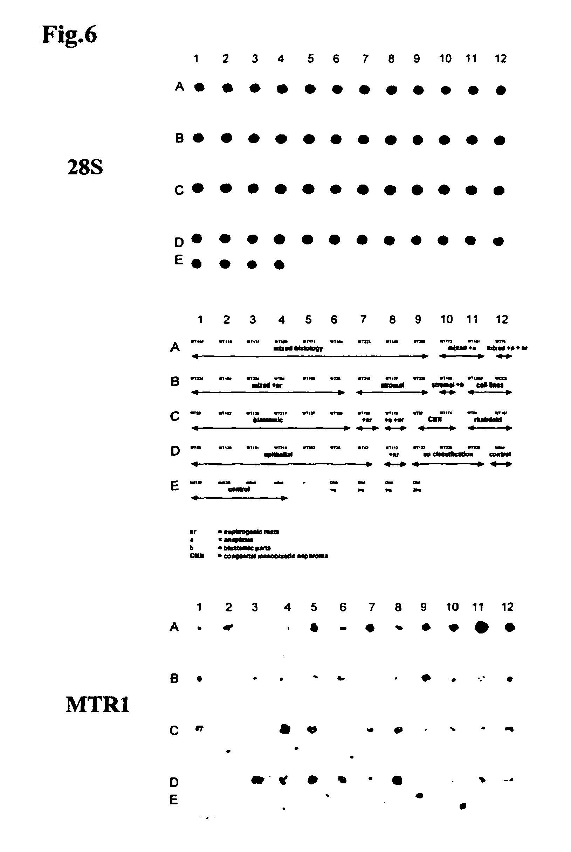

Figur 6: Hybridisierungssignale mit einer humanen MTR1-Sonde auf einem Northern-Dotblot mit 47 (alle heterogenen Wilms-Tumor-Gewebetynen repräsentierenden) Wilms-Tumoren und 4 nichterkrankten Nieren-Geweben.
   Alle WT wurden hinsichtlich WT1-Mutationen getestet. Das Beladungsschema für den Dotblot ist in der Mitte dargestellt. Jede Position enthält 2 µg Gesamt-RNA. Zum Nachweis der gleichmäßigen Beladung mit jeder Probe wurde eine Hybridisierung mit einer 28S-RNA-Sonde durchgeführt (oben). Zum Ausschluß von Hybridisierungssignalen auf der DNA-Ebene wurden vier DNA-Sonden (1 - 25 ng) ebenfalls auf die Filter aufgetüpfelt (Positionen E6-E9). In einer Reihe von WT wurden starke Signale beobachtet (Positionen A5,7,9-12, B1,9, C4,5,7,8 und D3-6,7). Im Durchschnitt waren die erhaltenen Signale höher als in normalem Gewebe. Die Signale wurden nach dem Zufallsprinzip mittels RT-PCR der Tumorproben bestätigt. Keine der WT-Zellinien (WT128c1, WCCS Position B11-12) zeigten in der RT-PCR ein Signal. Wt188 und Wt128c1 haben eine LOH für 11p und zeigen keine oder nur eine schwache Transkription von MTR1. Wt127 (Wt127 und Wt128cl besitzen eine WT1-Mutation) zeigen nur mäßige Mengen an MTR1-Transkripten.

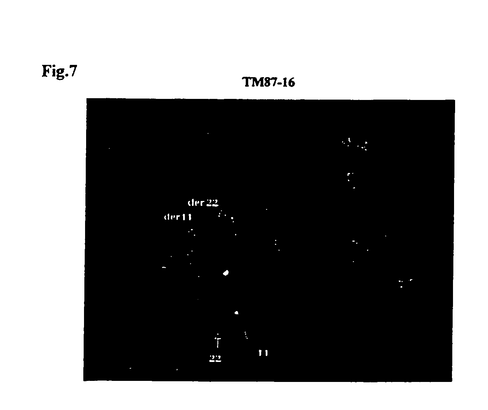

Figur 7: Zweifarbige FISH-Analyse von Metarphase-Spreitungen von der rhabdoiden Tumorzellinie TM87-16
   Rote Signale entsprechendem Chromosom 11 alpha-Satelliten und grüne Signale dem genomischen humanen MTR1-Bereich einschließlich des vermuteten Promotorbereichs am 5'-Ende von MTR1. Die Zellinie zeigt eine reziproke Translokation (t(11;22) (p15.5; q11.23)), wobei die Chromosom 11-Bruchstelle in dem genomischen Bereich liegt, der von dem PAC-Clon pDJ915F1 umspannt wird, derselbe Clon, der MTR1 enthält. Hybridisierungssignale wurden nur mit dem intakten Chromosom 11 und dem veränderten Chromosom 22 beobachtet, was eine vollständige Translokation von MTR1 auf das dadurch veränderte Chromosom 22 ohne Unterbrechung des Gens zeigt.

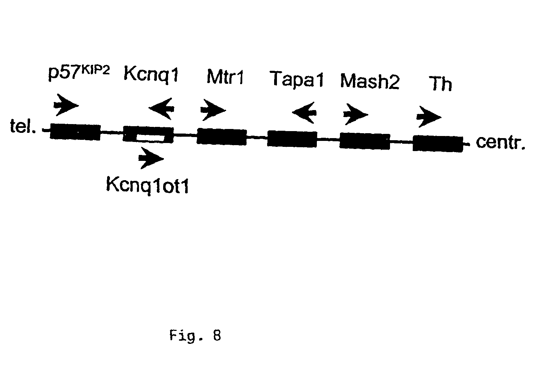

Fig. 8: Genomische Organisation des distalen Chromosoms 7 (bei 69,0 cM) der Maus
   Die relative Ordnung der Gene ist gezeigt vom Telomer (links) zum Centromer (rechts) und ihre transkriptionale Orientierung ist durch schwarze Pfeile angegeben.

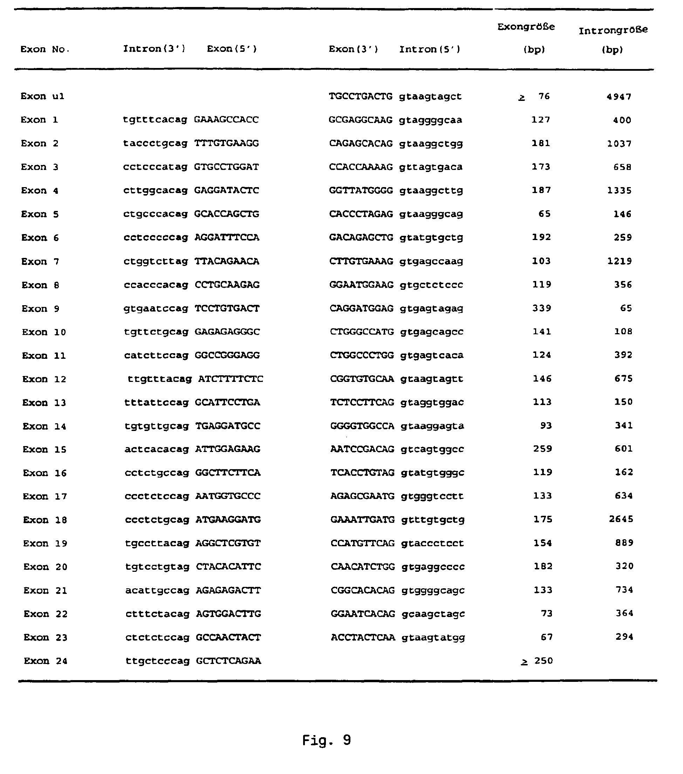

Fig. 9: Exon/Intron-Übergänge des Maus MTR1-Gens
   Exon-Sequenzen sind in Großbuchstaben, Intron-Sequenzen in Kleinbuchstaben angegeben. Spleißstellen sind in Fettdruck markiert. Alle Spleißstellen zeigen die üblichen AG-Exon-GT Splice-Akzeptor/Splice-Donor-Sequenz, außer der Übergang von Exon 22 zum Intron (AG-Exon-GC), was der humanen MTR1 Exon/Intron-Struktur entspricht. Das erste Exon ist u1 genannt, weil es keine Teile des in Exon 1 startenden offenen Leserahmens enthält.

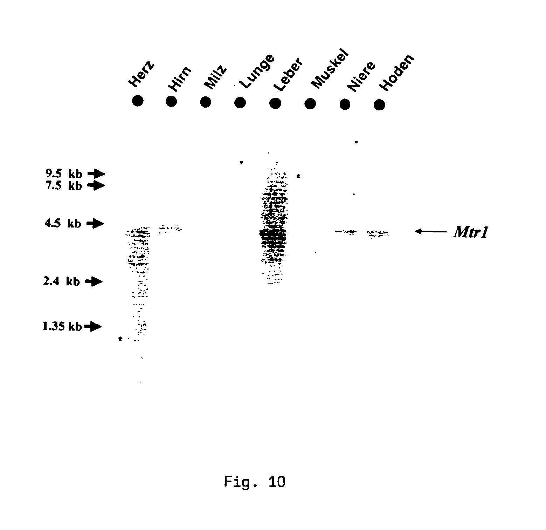

Fig. 10: Northern Blot mit Maus MTR1-Gen
   Northern Blot-Hybridisierung von Maus MTR1 mit einer Vielzahl adulter Gewebe. Der Northern Blot zeigt ein Signal mit einer Größe von ungefähr 4,4 kb. Die Fragmentpositionen eines Größenmarkers sind angegeben. Übermäßige Expression kann in Herz, Leber und Niere gesehen werden. Die Hybridisierungssonde stammte vom 3'-Ende des Maus-MTR1-Gens.

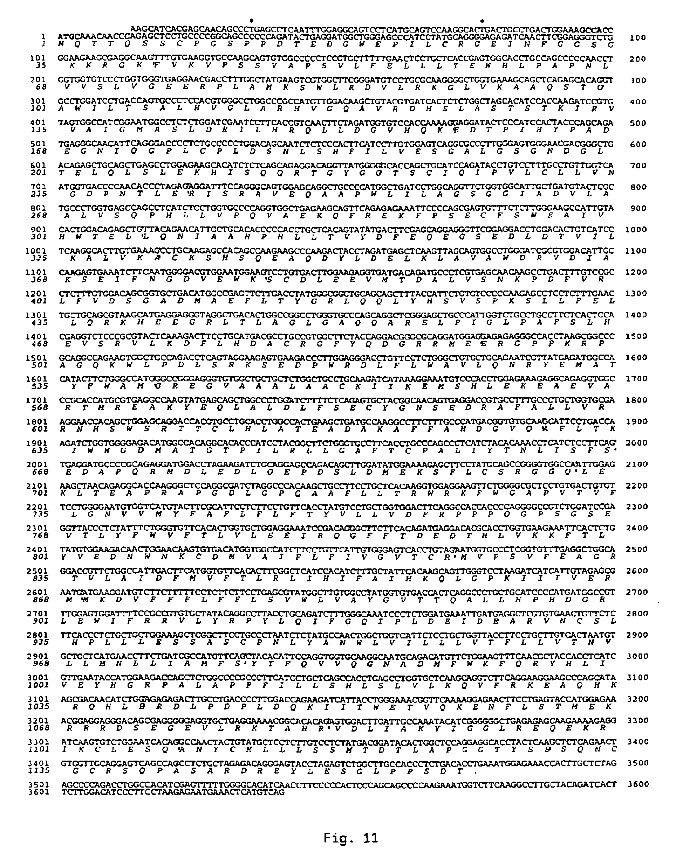

Fig. 11: cDNA-Sequenz von Maus MTR1 und abgeleitete Aminosäuresequenz
   Die cDNA-Sequenz und die vorgeschlagene Aminosäuresequenz von Maus MTR1 sind angegeben. Exonübergänge werden durch vertikal gestrichelte Linien ausgedrückt. Die nahezu ideale Translationsinitiations-Konsensussequenz ist in Fettdruck angegeben. Zwei "in-frame" Stopcodons in der 5'-UTR sind durch Sternchen markiert.

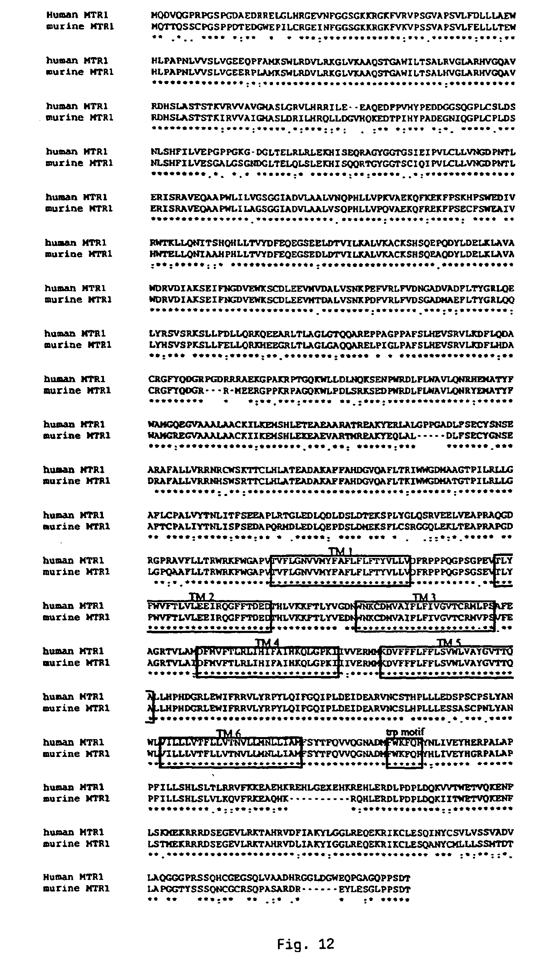

Fig. 12: Vergleich der humanen und der Maus-MTR1-Aminosäuresequenz
   Die menschliche und die Maus-MTR1-Sequenz wurden miteinander verglichen. Identität zwischen beiden Sequenzen wurde durch Sternchen unterhalb der Vergleichsreihen angegeben. Semikonservative Austausche wurden durch Einzelpunkte und konservative Austausche durch Doppelpunkte angegeben. Transmembrandomänen (TM1-6) und das Motiv für die Trp-Genfamilie sind in Kästchen gezeigt. Sie sind komplett identisch außer für den konservativen Austausch in TM1 (L --> V). Die Gesamthomologie der beiden Proteine ist 89,5%.

Die folgenden Beispiele erläutern die Erfindung.

### Beispiel 1: Allgemeine Verfahren

### (A) RT-PCR-Analyse

2 µg Gesamt-RNA wurden nach Spaltung mit DNase I mittels 250 pmol oligo(dT)-Primern und 300 E MMLV-Reverse-Transkriptase (Gibco BRL, Eggenstein, Deutschland) in einem Gesamtvolumen von 40 µl revers transkribiert. 2 µl dieses Reaktionsgemischs wurden in einer 20 µl PCR-Reaktion unter Verwendung der folgenden Genspezifischen Primer analysiert: MTR1E15F (5'-GTGCTGTCTTCCTGCTCAC-3'), MTR1E16R (5'-TGACACCCACGATGAACAGG-3'), MTR1E17F (5'-GGACTTCATGGTGTTCACGC-3'), MTR1E21R (5'-CGTGGTACTCCACAATCAGG-3'), MTR1E1F (5'-CCATGCAGGATGTCCAAGGC-3') und MTR1E24R (5'-TCAGGCAACACAAGTCAGG-3') [Anmerkung zur Namensgebung: z.B. MTR1E21R = stammt aus der cDNA von **MTR1** aus dem durchnummerierten Exon **21**, in der in der cDNA aufgelisteten Basenfolge (F) oder dazu revers komplementär (**R**)]. Die Primerpaare MTR1E15F, MTR1E16R und MTR1E17F und MTR1E21R wurden zum Nachweis der Transkription des MTR1-Gens in revers transkribierter mRNA unter folgenden PCR-Bedingungen verwendet: Einleitende Denaturierung (3 min, 94°C), 35 Zyklen (Denaturierung: 1 min, 94°C; Primeranlagerung: 1 min, 58°C; Primerverlängerung: 1 min, 72°C) und ein abschließender "Polishing"-Schritt (7 min, 72°C), 1,5 mM MgCl₂, 25 pmol von jedem Primer und 5 E Taq-Polymerase. Die Primerpaare MTR1E1F, MTR1E16R und MTR1E15F, MTR1E24R wurden zur Amplifikation des vollständigen offenen Leserahmens in zwei überlappenden Fragmenten verwendet, wobei das "Expand^{™} PCR System" entsprechend den vom Hersteller angegebenen Bedingungen (Roche Diagnostics, früher: Boehringer Mannheim, Deutschland) verwendet wurde.

### (B) Southern- und Northern-Analysen

Genomische DNA und Gesamt-RNA wurden nach Standard-Verfahren (Proteinase K/Phenol-Behandlung und Verwendung von "RNeasy"-Säulen (Qiagen, Hilden, Deutschland)) präpariert. Für Southern-Blots wurden 10 µg genomische DNA mit den erforderlichen Mengen der jeweiligen Restriktionsendonuclease vollständig gespalten und danach auf einem 0,8% Agarosegel elektrophoretisch aufgetrennt. Danach wurde die DNA mit 0,25 M HCl depuriniert und 30 min mit 0,5 N NaOH/ 1,5 M NaCl denaturiert. Der Transfer von DNA-Fragmenten und RNA auf "Hybond N+"-Membranen (Amersham, Braunschweig) erfolgte in 10xSSC für 48 Stunden, danach erfolgte eine UV-Quervernetzung. Northern-Dotblots wurden durch das Auftropfen von jeweils 2 µg Gesamt-RNA in einer symetrischen Anordnung auf eine Nylon-Membran (Hybond N+, Amersham) , an das sich eine UV-Quervernetzung anschloß, hergestellt. Außerdem wurden für eine ausführlichere Analyse der MTR1-Expression Northern-Blots mit RNAs aus einer Reihe von verschiedenen menschlichen fötalen Geweben (Clontech, Palo Alto, CA, USA) verwendet. Komplementäre DNA-Sonden des MTR1-Gens wurden mittels PCR amplifiziert und mit dem "Qiaquick"-Gelextraktions-Kit (Qiagen, Hilden, Deutschland) entsprechend den Angaben des Herstellers Gel-gereinigt und mit ³²P unter Verwendung eines Standard-Protokolls für "nick-translation" radioaktiv markiert. Die Hybridisierung aller Blots erfolgte 1 bis 2 Stunden bei 68°C in einer "Expresshyb^{™}"-Lösung (Clontech, Palo Alto, CA, USA) unter Verwendung von 2 x 10⁶ cpm/ml Sonde. Die Waschschritte nach der Hybridisierung erfolgten gemäß den Vorschlägen des Herstellers der "Expresshyb^{™}"-Lösung (Clontech, Palo Alto, CA, USA). Die Autoradiographie wurde 16 bis 120 Stunden mit Verstärkerfolien bei -80°C durchgeführt.

### (C) Zellinien

Somatische Zellhybride, die ein väterliches (GM10927B, GM1944, GM11941) oder mütterliches Chromosom 11 (GM07300, GM13400) in einem Nager-Hintergrund enthielten, wurden wie kürzlich beschrieben gehalten (Gabriel et al., PNAS 95 (1998), 14857-14862). Die rhabdoide Zellinie TM87-16 (Karnes et al., Genet. Cytogenet. 56 (1991), 31-38) wurde in DMEM gehalten, das mit 10% hitzeinaktiviertem fötalem Kälberserum (FCS), Pen/Strep und Glutamin supplementiert war.

### (D) Bestimmung der allelen Expression

cDNAs der somatischen Zellhybride, die entweder das väterliche oder mütterliche humane Chromosom 11 oder Maus Chromosom 7 enthielten, wurden mittels der in (A) vorstehend beschriebenen Primer und Amplifikationsprotokolle amplifiziert. Die PCR-Produkte wurden auf 2% Agarosegelen aufgetrennt und auf eine Membran wie in (B) vorstehend beschrieben transferiert. Produkte wurden durch Hybridisierung mit einer ³²P-markierten cDNA-Sonde verifiziert und ein PCR-Produkt wurde direkt sequenziert. Für alle Primersätze wurden Kontrollamplifikationen hinsichtlich möglicher Kontaminationen unter identischen PCR-Bedingungen durchgeführt, allerdings ohne reverse Transkription der präparierten RNA.

### (E) FISH-Analyse

Fluoreszenz-in situ-Hybridisierung (FISH) an Metaphase-Chromosomen wurde wie kürzlich beschrieben durchgeführt (Spangenberg et al., Genomics 48 (1998), 178-185; Winterpacht et al., Cytogenetics and Cell Genetics 75/2-3/96 (1996), 132-135). Metaphase-Spreitungen von TM87-16-Zellen wurden wie kürzlich beschrieben durchgeführt (Löbbert et al., Genes Chromosomes Cancer 21 (1998), 347-350; Spangenberg et al., Genomics 48 (1998), 178-185; Winterpacht et al., Cytogenetics and Cell Genetics 75/2-3/96 (1996), 132-135). Genomische, das MTR1-Gen umfassende Fragmente wurden mit flankierenden Primern mittels des "Expand^{™}"-PCR-Systems (Roche Diagnostics, früher: Boehringer Mannheim, Deutschland) entsprechend den Angaben des Herstellers amplifiziert. Diese Fragmente wurden mit "DIG-11"-dUTP entsprechend Boehringer Mannheim-Protokollen "nick-translatiert" und über Nacht bei 37°C in einer Feuchtkammer hybridisiert. Die Waschschritte nach der Hybridisierung wurden bei 42°C in 50% Formamid, 1 x SSC dreimal jeweils 5 min und in 0,3 x SSC dreimal jeweils 5 min durchgeführt. Der immunologische Nachweis der hybridisierten Sequenzen wurde mittels eines Maus-anti-DIG-Antikörpers (Boehringer Mannheim, Deutschland) durchgeführt, der 1:1000 in 4 x SSC, 0,1% Tween 20 verdünnt worden war und "TRITC"-markiertem anti-Maus-IgG aus Kaninchen (Boehringer Mannheim, Deutschland), des 1:100 verdünnt worden war. Es schloß sich eine Signalamplifikation mit "TRITC"-markiertem 1:64 verdünnten anti-Kaninchen-IgG (Boehringer Mannheim, Deutschland) an. Chromosomen wurden mit 4'-6-Diamidino-2-Phenylindol (DAPI) gegengefärbt und mit "Vectashield^{™}" (Vector Laboratories, Burlingame, CA, USA) bedeckt. Die FISH wurde mittels einer Hochleistungs-CCD-Kamera (Applied Imaging, Santa Clara, CA., USA) aufgenommen und mit der "Cytovision 2.21"-Software (Applied Imaging, Santa Clara, CA, USA) ausgewertet.

### (F) Herstellung und Reinigung eines erfindungsgemäßen Proteins (MTR1)

Die DNA von Fig. 4 (ohne Exon 18) wird mit BamHI-Linkern versehen, mit BamHI nachgespalten und in den mit BamHI gespaltenen Expressionsvektors pQE-8 (Qiagen) inseriert. Es wird das Expressionsplasmid pQE-8/MRT erhalten. Ein solches kodiert für ein Fusionsprotein aus 6 Histidin-Resten (N-Terminuspartner) und dem erfindungsgemäßen [um Exon 18 verkürzten] MRT1-Protein von Fig. 4 (C-Terminuspartner). pQE-8/MRT wird zur Transformation von E.coli SG 13009 (vgl. Gottesman, S. et al., J. Bacteriol. 148, (1981), 265-273) verwendet. Die Bakterien werden in einem LB-Medium mit 100µg/ml Ampicillin und 25µg/ml Kanamycin kultiviert und 4 h mit 60µM Isopropyl-ß-D-Thiogalactopyranosid (IPTG) induziert. Durch Zugabe von 6 M Guanidinhydrochlorid wird eine Lyse der Bakterien erreicht, anschließend wird mit dem Lysat eine Chromatographie (Ni-NTA-Resin) in Gegenwart von 8 M Harnstoff entsprechend der Angaben des Herstellers (Qiagen) des Chromatographie-Materials durchgeführt. Das gebundene Fusionsprotein wird in einem Puffer mit pH 3,5 eluiert. Nach seiner Neutralisierung wird das Fusionsprotein einer 18 % SDS-Polyacrylamid-Gelelektrophorese unterworfen und mit Coomassie-Blau angefärbt (vgl. Thomas, J.O. und Kornberg, R.D., J.Mol.Biol. 149 (1975), 709-733).

Es zeigt sich, daß ein erfindungsgemäßes (Fusions)protein in hochreiner Form hergestellt werden kann.

### (G) Herstellung und Nachweis eines erfindungsgemäßen Antikörpers

Ein erfindungsgemäßes Fusionsprotein gemäß vorstehendem Abschnitt (F) wird einer 18% SDS-Polyacrylamid-Gelelektrophorese unterzogen. Nach Anfärbung des Gels mit 4 M Natriumacetat wird eine ca. 97 kD Bande aus dem Gel herausgeschnitten und in Phosphat gepufferter Kochsalzlösung inkubiert. Gel-Stücke werden sedimentiert, bevor die Proteinkonzentration des Überstandes durch eine SDS-Polyacrylamid-Gelelektrophorese, der eine Coomassie-Blau-Färbung folgt, bestimmt wird. Mit dem Gel-gereinigten Fusionsprotein werden Tiere wie folgt immunisiert:
a) Immunisierungsprotokoll für polyklonale Antikörper im Kaninchen
   Pro Immunisierung werden 35 µg Gel-gereinigtes Fusionsprotein in 0,7 ml PBS und 0,7 ml komplettem bzw. inkomplettem Freund's Adjuvans eingesetzt.
   Tag O: 1. Immunisierung komplettes Freund's Adjuvans)
   Tag 14: 2. Immunisierung (inkomplettes Freund's Adjuvans; icFA)
   Tag 28: 3. Immunisierung (icFA)
   Tag 56: 4. Immunisierung (icFA)
   Tag 80: Ausbluten

   Das Serum des Kaninchens wird im Immunoblot getestet. Hierzu wird ein erfindungsgemäßes Fusionsprotein von vorstehendem Abschnitt (F) einer SDS-Polyacrylamid-Gelelektrophorese unterzogen und auf ein Nitrocellulosefilter übertragen (vgl. Khyse-Andersen, J., J. Biochem. Biophys. Meth. 10, (1984), 203-209). Die Western Blot-Analyse wurde wie in Bock, C.-T. et al., Virus Genes 8, (1994), 215-229, beschrieben, durchgeführt. Hierzu wird das Nitrocellulosefilter eine Stunde bei 37°C mit einem ersten Antikörper inkubiert. Dieser Antikörper ist das Serum des Kaninchens (1:10000 in PBS). Nach mehreren Waschschritten mit PBS wird das Nitrocellulosefilter mit einem zweiten Antikörper inkubiert. Dieser Antikörper ist ein mit alkalischer Phosphatase gekoppelter monoklonaler Ziege Anti-Kaninchen-IgG-Antikörper (Dianova) (1:5000) in PBS. Nach 30-minütiger Inkubation bei 37°C folgen mehrere Waschschritte mit PBS und anschließend die alkalische Phosphatase-Nachweisreaktion mit Entwicklerlösung (36µM 5' Bromo-4-chloro-3-indolylphosphat, 400µM Nitroblau-tetrazolium, 100mM Tris-HCl, pH 9.5, 100 mM NaCl, 5 mM MgCl₂) bei Raumtemperatur, bis Banden sichtbar werden.
   Es zeigt sich, daß erfindungsgemäße, polyklonale Antikörper hergestellt werden können.
b) Immunisierungsprotokoll für polyklonale Antikörper im Huhn
   Pro Immunisierung werden 40µg Gel-gereinigtes Fusionsprotein in 0,8 ml PBS und 0,8 ml komplettem bzw. inkomplettem Freund's Adjuvans eingesetzt.
   Tag O: 1. Immunisierung (komplettes Freund's Adjuvans)
   Tag 28: 2. Immunisierung (inkomplettes Freund's Adjuvans; icFA)
   Tag 50: 3. Immunisierung (icFA)

   Aus Eigelb werden Antikörper extrahiert und im Western Blot getestet. Es werden erfindungsgemäße, polyklonale Antikörper nachgewiesen.
c) Immunisierungsprotokoll für monoklonale Antikörper der Maus
   Pro Immunisierung werden 12µg Gel-gereinigtes Fusionsprotein in 0,25 ml PBS und 0,25 ml komplettem bzw. inkomplettem Freund's Adjuvans eingesetzt; bei der 4. Immunisierung ist das Fusionsprotein in 0,5 ml (ohne Adjuvans) gelöst.
   Tag O: 1. Immunisierung (komplettes Freund's Adjuvans)
   Tag 28: 2. Immunisierung (inkomplettes Freund's Adjuvans; icFA)
   Tag 56: 3. Immunisierung (icFA)
   Tag 84: 4. Immunisierung (PBS)
   Tag 87: Fusion

   Überstände von Hybriddmen werden im Western Blot getestet. Erfindungsgemäße, monoklonale Antikörper werden nachgewiesen.

### Beispiel 2: Transkripte, alternatives Spleißen und genomische Struktur von MTR1

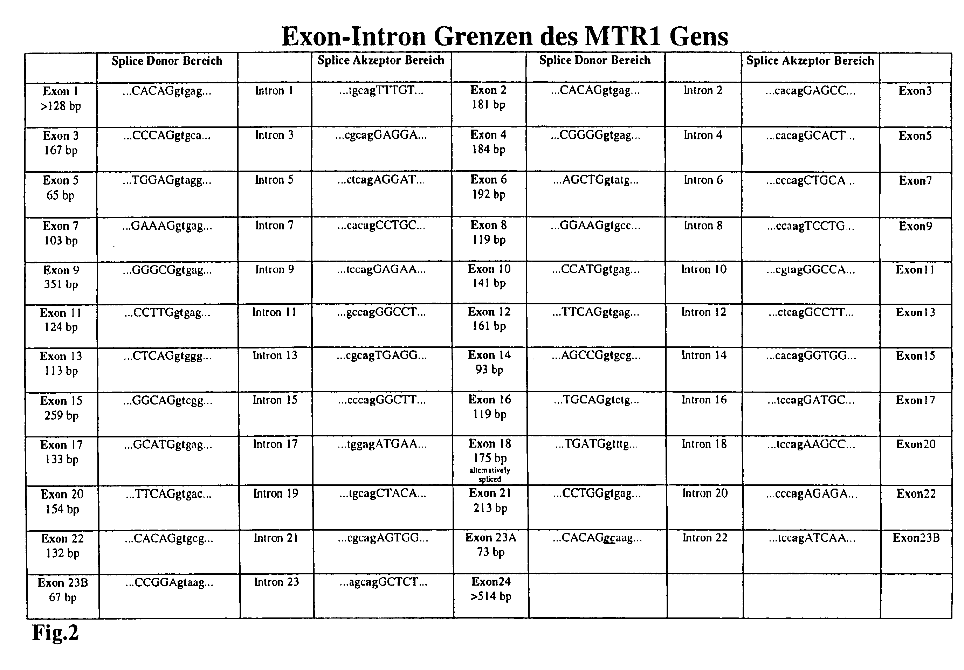

Die Analyse der genomischen humanen MTR1-Teilregion ergab 24, den ORF (Größe etwa 3,9 kb) umfassende Exons und eine Gesamtlänge von 18,5 kb. Die Lokalisation von MTR1 ist proximal zu TSSC4 und distal zum ersten 5'-Exon des KvLQT1-Gens. Somit liegt das neue Gen innerhalb von BWSCR1 auf dem menschlichen Chromosom 11. Um direkt nachzuweisen, daß die vorhergesagten Exons in mRNA transkribiert werden, wurde zur Amplifikation von überlappenden Genbereichen "Exon-Verbindungs"-Experimente durchgeführt. Da BWSCR1 sowohl an BWS als auch WTs beteiligt ist, wurden cDNA von humanen fötalen Nieren und Nieren von Erwachsenen erzeugt. Da die Amplifikationsprodukte Introns umfaßten, konnten von transkribierten Sequenzen stammende PCR-Produkte von PCR-Produkten aus nicht-transkribierter genomischer DNA unterschieden werden. Der Minimalsatz an Primern, der für die Abdeckung des MTR1-ORF notwendig war, waren MTR1E1F/MTR1E16R und MTR1E15R/MTR1E24R. Mit reverser Transkription gekoppelte PCR-Reaktionen (RT-PCR) von RNA-Präparationen aus fötalen Nieren und Nieren von Erwachsenen ergab Produkte mit der erwarteten Größe. Die PCR-Produkte wurden subkloniert und beide Stränge sequenziert. Nur ein Exon (Exon 19) konnte bisher nicht identifiziert werden. Jedoch konnte ein zusätzliches Exon reproduzierbar in dem RT-PCR-Produkt nachgewiesen werden (Exon 23A). Außerdem erzeugte das Primerpaar MTR1E15F/MTR1E24R zwei PCR-Produkte aus der verwendeten cDNA, die sich in einem Exon (Exon 18) unterschieden. Man kann daher davon ausgehen, daß dieses Exon einem alternativen Spleißen unterliegt. Mittels des Vergleichs der genomischen Sequenz mit dem MTR1-Transkript konnten die genauen Exon/Intron-Grenzen des MTR1-Gens bestimmt werden (Figur 2).

### Beispiel 3: Expression des MTR1-Gens

Die Amplifikationsprodukte des Primerpaars MTR1E15R/MTRE24R wurden gelgereinigt und zur Bestimmung des Expressionsprofils und der Transkriptgröße des MTR1-Gens auf Northern-Blots mit RNAs aus unterschiedlichen Geweben (MTN) verwendet. Um Kreuzhybridisierung mit Genen aus der TRP-Genfamilie ausschließen zu können, wurden Southern-Blots mit geschnittener genomischer DNA und genomischem pDJ915F1 (genomischer PAC-Klon; erhältlich von Resourcenzentrum des Dt. Humangenomprojekts (RZPD), Berlin) unter identischen Bedingungen hybridisiert. In den Southern-Blots wurden sowohl in den genomischen DNA-Fragmenten als auch in den Fragmenten des PAC-Klons identische Signale erhalten. Somit war die Hybridisierung spezifisch und es zeigte sich kein Hinweis auf eine Kreuzhybridisierung. Ein 4,5 kb Transkript konnte in einer Reihe von fötalen und erwachsenen Geweben nachgewiesen werden (Figur 3A). Da die experimentell bestätigten Exons nur 3913 bp umfassen, deuten diese Ergebnisse darauf hin, daß etwa 500 bp nicht-translatierter RNA noch zu identifizieren sind. Auf Northern-Blots zeigt das MTR1-Gen eine signifikante Expression in fötaler Leber, Niere und Milz sowie in Niere, Leber, Dünndarm, Lunge, Pankreas, Colon, Prostata und Drüsengewebe von Erwachsenen (Figur 3B). Dieses Ergebnis zeigt somit, daß MTR1 in einer großen Zahl von Geweben exprimiert wird. Da sich die beiden vermeintlichen Spleißformen von MTR1 nur in Exon 18 (175 bp) unterscheiden, können diese beiden Transkripte auf Northern-Blots nicht unterschieden werden.

### Beispiel 4: Proteinstruktur

Aufgrund des alternativ gespleißten Exons 18 kann man von wenigstens zwei humanen Isoformen ausgehen. Exon 1 enthält eine fast ideale "Kozak-consensus"-Sequenz, was nahelegt, daß die Initiation der Translation an dieser Position einsetzt. Der längste ORF mit diesem Initiationscodon (einschließlich Exon 18) umfaßt 3495 bp, die ein Polypeptid mit 1165 Aminosäuren codieren und 6 Transmembrandomänen enthält (Figur 4). Das theoretische Molekulargewicht ist 131,45 kDa. Die zweite Spleißversion kodiert einen kleineren ORF mit 872 Aminosäuren (Figur 4). Dieses Protein hat die ersten 869 Aminosäuren mit der längeren MTR1-Version gemeinsam, besteht somit aus 4 - 5 Transmembran-Domänen. Das theoretische Molekulargewicht beträgt 97,74 kDa. Es wurde gefunden, daß die MTR1-Proteine in der Plasmamembran lokalisiert sind, wobei der C-Terminus innerhalb der Zelle liegt.

### Beispiel 5: Väterliche Expression von MTR1-mRNA

Wie bereits kürzlich beschrieben, wird die bevorzugte monoallelische Expression "imprinteder Gene" (= ein Gen wird nur von einem der parentalen Allele transkribiert, wobei das von dem zweiten Elternteil stammende Allel durch DNA-Modifikationen, z.B. Methylierungen, stillgelegt ist) in somatischen, ein einzelnes humanes Chromosom 11 enthaltenden Zellhybriden aufrechterhalten (Gabriel et al., PNAS 95 (1998), 14857-14862; Mitsuya et al., Hum. Mol. Genet. 8 (1999), 1209-1217). Dies ermöglicht die Verwendung von Zellhybriden, die entweder einen vom Vater oder der Mutter stammenden humanen chromosomalen 11p15-Bereich enthalten, als Modellsystem zur Identifizierung "geprägter" Gene aus dem BWSCR1-Bereich.

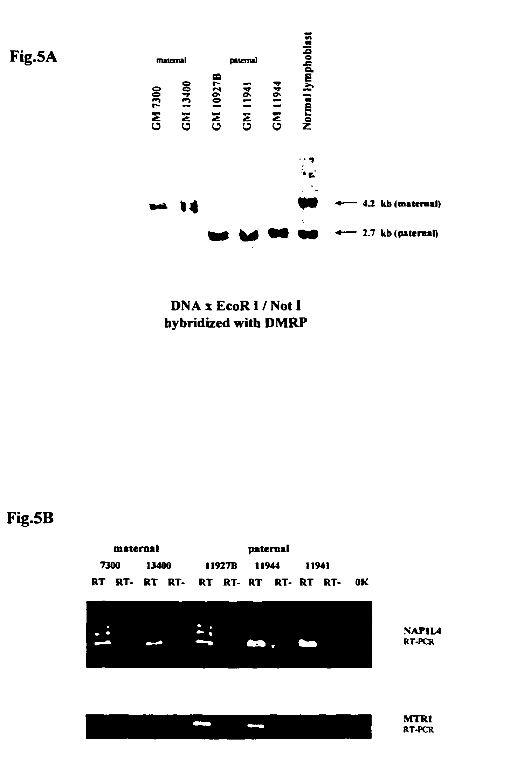

Die Expression des MTR1-Gens wurde mittels RT-PCR in einem Teilsatz der somatischen Zellhybride durchgeführt, die kürzlich von Gabriel et al. (PNAS 95 (1998), 14857-14862) beschrieben worden waren. Zu diesen zählten zwei Zellinien mit einem von der mütterlichen Seite stammenden humanen Chromosom 11 (GM7300, GM13400) und drei somatische Zellhybride mit einer väterlichen Kopie von 11p15 (GM10927B, GM11944, GM11941). Zur Überprüfung der Beibehaltung der epigenetischen Chromatinstadien wurden die Hybride hinsichtlich einer unterschiedlichen Methylierung an einer kürzlich beschriebenen mütterlich methylierten NotI-Restriktionsstelle innerhalb des KvQT1-Genbereichs (Gabriel et al., PNAS 95 (1998), 14857-14862; Mitsuya et al., Hum. Mol. Genet. 8 (1999), 1209-1217; Smilinich et al., PNAS 96 (1999), 8064-8069) analysiert. Alle untersuchten Zellhybride zeigten die Methylierung entsprechend ihrer väterlichen Herkunft (Figur 5A, mütterliches Hybridisierungssignal bei 4,2 kb, väterliches bei 2,7 kb), wodurch die erwarteten Epigenesis-Chromatinstadien bestätigt wurden. Zur Verifizierung der Integrität der 11p15.5-Transkripte wurden die Zellhybride hinsichtlich der Anwesenheit eines codierenden Fragments von NAP1L4 (= Nucleosome-assemblyprotein **1** like gene No. **4** aus 11p15.5, welches biallelisch transkribiert wird) als ein bi-allelisches Transkript untersucht. Figur 5B zeigt die für das vorstehend erwähnte Genfragment erhaltenen RT-PCR-Produkte, d.h. zeigt die Anwesenheit von 11p15.5 in den untersuchten Hybriden.

Die drei, das von der väterlichen Seite stammende Chromosom 11 enthaltenden somatisches Zellhybride zeigten ein MTR1-RT-PCR-Produkt (GM11941 nur im Anschluß an Southern-Blot-Hybridisierung), während bei Hybriden, die eine mütterliche Kopie von Chromosom 11 enthielten, keine Produkte gefunden werden konnten. Der Befund, daß die in diesem Produkt erhaltenen PCR-Produkte von MTR1 stammten, wurde durch Hybridisierung des geblottetenen Gels mit einer radioaktiv markierten MTR1-Sonde und durch direkte Sequenzierung eines der PCR-Produkte bestätigt. Die Daten ergaben, daß das MTR1-Gen (bevorzugt) väterliche Expression zeigt.

### Beispiel 6: Expression des MTR1-Gens in Tumoren

Etwa 5 - 15% von Wilms Tumoren (WT) beruhen auf Mutationen innerhalb des Wilm-Tumor-Gens 1 (WT1), das auf dem chromosomalen Bereich 11p13 lokalisiert ist und einen einen Zinkfinger enthaltenden Transkriptionsfaktor kodiert. WT sind heterogen mit variablem Gehalt an blastemischem, stromalem oder epithelialem Gewebe. Es wurde daher eine repräsentative Anzahl von 47 WT ausgewählt, einschließlich von zwei Fällen mit LOH auf Chromosom 11p (WT128c1, WT188) und zwei WT-Zellinien (WT128c1, WCCS). Jeder Tumor wurde histologisch charakterisiert und hinsichtlich WT1-Mutationen analysiert. WT128 und WT127 waren die einzigen WT, die eine Mutation in WT1 hatten (WT128 mit einem vorzeitigen Stopcodon, veröffentlicht in Loebbert et al., Genes Chromosomes Cancer 21 (1998), 347-350; WT127 mit einem vorzeitigen Stopcodon in Zinkfinger 2 (Exon 8)). Gleiche Mengen an Gesamt-RNA (2 µg) aus 47 Tumoren, 5 nicht betroffenen Kontrollgeweben (fötale Niere und Niere von Erwachsenen) und 4 DNA-Sonden mit unterschiedlichen Konzentrationen (1 - 25 ng) wurden zur Herstellung eines RNA-Dotblots verwendet. Nach Hybridisierung mit einem radioaktiv markierten MTR1 cDNA-Fragment konnten in beinahe allen WT-Proben Signale in unterschiedlicher Stärke nachgewiesen werden. Durchschnittlich waren die Signale im Vergleich zu denen in den normalen Geweben höher. Hybridisierungssignale wurden nach dem Zufallsprinzip mittels RT-PCR an den Tumor-Proben mit den Primerpaaren MTR1E115F/MTR1E16R und MTR1E17F/MTR1E21R bestätigt. Das letztere Primerpaar umspannt das alternativ gespleißte Exon 18. Alle RT-PCR-Reaktionen, die mit diesen Primern eine Amplifikation ergaben, zeigten beide Spleißversionen von MTR1. Beide WT-Zellinien zeigten kein MTR1-Signal nach Hybridisierung oder nach Analyse der RNA mittels RT-PCR. Außerdem wurden drei Tumor-Proben von BWS-Patienten (ohne WT1-Mutation) analysiert, die geringe Mengen des MTR1-Transkripts zeigten.

Da Veränderungen in 11p15.5, insbesondere in BWSCR1, nicht nur eine Korrelation mit der Entwicklung mit Rhabdomyosarkomen zeigen (Karnik et al., Hum. Mol. Genet. 7 (1998), 895-903), wurden Gewebeproben von drei Rhabdomyosarkomen in die Untersuchung miteinbezogen. RT-PCR mit den vorstehend erwähnten Primerpaaren ergab Amplifikation, was darauf hinweist, daß MTR1 auch in diesem Tumortyp exprimiert wird.

### Beispiel 7: Translokation des MTR1-Gens in einem rhabdoiden Tumor (TM87-16)

Maligne rhabdoide Tumore (MRT) gehören zu der Tumorgruppe von Sarkomen der Weichteile und sind außergewöhnlich aggressiv mit einem hohen metastatischen Potential. Es wurde zuerst davon ausgegangen, daß es sich dabei um Wilms-Tumor-Varianten handelt, da sie jedoch an zahlreichen Stellen außerhalb des Nierengewebes beobachtet wurden, geht man inzwischen davon aus, daß es sich um einen anderen Tumortyp handelt. Karnes et al. (Cancer Genet. Cytogenet. 56 (1991), 31-38) etablierten eine Zellinie (TM87-16) aus einem Pleuraerguß einer retroperitonealen Masse, der in einem 21 Monate alten männlichen Kaukasier diagnostiziert worden war. Die Chromosomenanalyse ergab eine reziproke Translokation t(11;22) (p15.5;q11.23), deren Bruchstelle in der von dem PAC-Clon pDJ915F1 (der MTR1 enthält) umspannten genomischen Region lokalisiert ist. Da die genaue Bruchstelle noch nicht bestimmt worden war, wurden genomische Fragmente von MTR1 amplifiziert und danach in einer FISH-Analyse an TM87-16 Metaphase-Spreitungen verwendet. Das FISH-Experiment zeigte, daß MTR1 ohne Unterbrechung auf das Chromosom 22 transloziert worden war (Figur 7). Somit ist die Translokations-Bruchstelle in TM87-16 proximal zu MTR1. Die RT-PCR-Analyse von Gesamt-RNA aus TM87-16 mit den Primern MTR1E15F/MTR1E16R zeigte eine mäßige Expression des MTR1-Gens.

Folgende Klone wurden bei der DSMZ (Deutsches Sammlung von Mikroorganismen und Zellkulturen GmbH, Mascheroder Weg 1b, 38124 Braunschweig) am 19. Oktober 1999 gemäß Budapester Vertrag hinterlegt:
E. coli MTR1-17/21 DSM 13108
E. coli MTR1-1/24 DSM 13107

MTR1-17/21 beinhaltet die Exons 17-21 inkl. dem alternativ gespleißten 18 des Gens humanen MTR1. MTR-1/24 beinhaltet die Exons 1 bis 24 (inkl.) ohne das Exon 18 des humanen MTR1-Gens (gesamter ORF).

## Patentansprüche

1. cDNA-Sequenz, die für ein Protein (MTR1) der Trp (transient receptor potential) Familie mit einer der in Figur 4 gezeigten Aminosäuresequenzen codiert.

2. cDNA-Sequenz nach Anspruch 1, die eine der in Figur 4 gezeigten DNA-Sequenzen umfasst.

3. cDNA-Sequenz nach Anspruch 1 oder 2, wobei das Protein an der Ca²⁺-Regulation innerhalb der Zelle beteiligt ist.

4. Ribozym, **dadurch gekennzeichnet, dass** es zu der cDNA-Sequenz nach einem der Ansprüche 1 bis 3 komplementär ist und an die von dieser cDNA-Sequenz transkribierte RNA spezifisch binden und diese spalten kann, wodurch die Synthese des von dieser cDNA Sequenz codierten Proteins verringert oder gehemmt wird.

5. Antisense-RNA, **dadurch gekennzeichnet, dass** sie zu der cDNA Sequenz nach einem der Ansprüche 1 bis 3 komplementär ist und an die von dieser cDNA-Sequenz transkribierte RNA spezifisch binden kann, wodurch die Synthese des von dieser cDNA-Sequenz codierten Proteins verringert oder gehemmt wird.

6. Expressionsvektor, die cDNA-Sequenz nach einem der Ansprüche 1 bis 3 oder die das Ribozym nach Anspruch 4 oder die Antisense RNA nach Anspruch 5 codierende DNA enthaltend.

7. Wirtszelle, die mit dem Expressionsvektor nach Anspruch 6 transformiert ist.

8. MTR1-Protein, das eine der in Fig. 4 gezeigten Aminosäuresequenzen umfasst.

9. Verfahren zur Herstellung eines MTR1-Proteins gemäß Anspruch 8, das die Züchtung der Wirtszelle nach Anspruch 7 unter geeigneten Bedingungen und die Gewinnung des Proteins aus der Zelle oder dem Zuchtmedium umfasst.

10. Antikörper oder Fragment davon, der (das) an das MTR1- Protein nach Anspruch 8 spezifisch bindet, wobei das MTR1-Protein aus einer der in Fig. 4 gezeigten Aminosäuresequenz besteht.

11. Arzneimittel, das eine cDNA-Sequenz nach einem der Ansprüche 1 bis 3, das Ribozym nach Anspruch 4, die Antisense-RNA nach Anspruch 5, den Expressionsvektor nach Anspruch 6, das MTR1- Protein nach Anspruch 8 oder den Antikörper oder das Fragment davon nach Anspruch 10 enthält.

12. Verwendung der cDNA-Sequenz nach einem der Ansprüche 1 bis 3, des Ribozyms nach Anspruch 4, der Antisense-RNA nach Anspruch 5, des Expressionsvektors nach Anspruch 6, des MTR1-Proteins nach Anspruch 8 oder des Antikörpers oder des Fragments davon nach Anspruch 10 zur Herstellung eines Arzneimittels zur Prävention oder Behandlung von Erkrankungen, die mit einer veränderten Expression der MTR1 codierenden DNA-Sequenz, einer veränderten Aktivität des MTR1-Proteins oder einer fehlerhaften Regulation des Ca²⁺-Eintritts in die Zellen assoziiert sind.

13. Diagnoseverfahren zum Nachweis einer eventuell gestörten MTR1-Expression, bei dem man eine Probe mit der cDNA-Sequenz nach einem der Ansprüche 1 bis 3 oder dem Antikörper oder des Fragments davon nach Anspruch 10 in Berührung bringt und direkt oder indirekt bestimmt, ob sich die Konzentration, Länge und/oder Sequenz des MTR1-Proteins gemäß Anspruch 8 oder codiert durch die cDNA-Sequenz gemäß Anspruch 1 oder 2 oder der dieses Protein codierenden mRNA im Vergleich zu einer Kontrollprobe unterscheiden.

14. Diagnostischer Kit zur Durchführung des Verfahrens nach Anspruch 13, der die cDNA-Sequenz nach einem der Ansprüche 1 bis 3 und/oder den Antikörper oder das Fragment davon nach Anspruch 10 enthält.

## Claims

1. A cDNA sequence which codes for a protein (MTR1) of the Trp (transient receptor potential) family with one of the amino acid sequences shown in figure 4.

2. The cDNA sequence according to claim 1, which comprises one of the DNA sequences shown in figure 4.

3. The cDNA sequence according to claim 1 or 2, wherein the protein is involved in the Ca²⁺ regulation within the cell.

4. A ribozyme, **characterized in that** it is complementary to the cDNA sequence according to any of claims 1 to 3 and can bind specifically to, and cleave, the RNA transcribed by this cDNA sequence so as to reduce or inhibit the synthesis of the protein encoded by this cDNA sequence.

5. An antisense RNA, **characterized in that** it is complementary to the cDNA sequence according to any of claims 1 to 3 and can bind specifically to the RNA transcribed by this cDNA sequence so as to reduce or inhibit the synthesis of the protein encoded by this cDNA sequence.

6. An expression vector containing the cDNA sequence according to any of claims 1 to 3 or the DNA coding for the ribozyme according to claim 4 or the antisense RNA according to claim 5.

7. A host cell which is transformed with the expression vector according to claim 6.

8. An MTR1 protein which comprises one of the amino acid sequences shown in figure 4.

9. A process for the production of an MTR1 protein according to claim 8, which comprises culturing the host cell according to claim 7 under suitable conditions and recovering the protein from the cell or the culture medium.

10. An antibody or fragment thereof, which binds specifically to the MTR1 protein according to claim 8, the MTR1 protein consisting of one of the amino acid sequences shown in figure 4.

11. A pharmaceutical preparation which contains a cDNA sequence according to any of claims 1 to 3, the ribozyme according to claim 4, the antisense RNA according to claim 5, the expression vector according to claim 6, the MTR1 protein according to claim 8 or the antibody or the fragment thereof according to claim 10.

12. Use of the cDNA sequence according to any of claims 1 to 3, of the ribozyme according to claim 4, of the antisense RNA according to claim 5, of the expression vector according to claim 6, of the MTR1 protein according to claim 8 or of the antibody or the fragment thereof according to claim 10 for the production of a medicament for the prevention or treatment of diseases which are associated with a modified expression of the MTR1 encoding DNA sequence, a modified activity of the MTR1 protein or a misregulation of the Ca²⁺ entry into the cells.

13. A diagnostic method for the detection of a possibly disturbed MTR1 expression, wherein a sample is contacted with the cDNA sequence according to any of claims 1 to 3 or the antibody or the fragment thereof according to claim 10 and determined directly or indirectly as to whether the concentration, length and/or sequence of the MTR1 protein according to claim 8 or encoded by the cDNA sequence according to claim 1 or 2 or the mRNA encoding this protein differs from a control sample.

14. A diagnostic kit for carrying out the method according to claim 13, which contains the cDNA sequence according to any of claims 1 to 3 and/or the antibody or the fragment thereof according to claim 10.

## Revendications

1. Séquence de cADN codant une protéine (MTR1) de la famille Trp (transient receptor potential = potentiel récepteur transitoire) avec l'une des séquences d'acide aminé illustrées à la figure 4.

2. Séquence de cADN selon la revendication 1 comprenant l'une des séquences d'ADN illustrées à la figure 4.

3. Séquence de cADN selon la revendication 1 ou 2, la protéine participant à la régulation de Ca²⁺ dans la cellule.

4. Ribozyme, **caractérisé par le fait qu'**il est complémentaire à la séquence de cADN selon l'une des revendications 1 à 3 et qu'il peut se lier de manière spécifique à l'ARN transcrit par cette séquence de cADN et diviser ce dernier, d'où la synthèse de la protéine codée par cette séquence de cADN est réduite ou inhibée.

5. ARN anti-sens, **caractérisé par le fait qu'**il est complémentaire à la séquence de cADN selon l'une des revendications 1 à 3 et qu'il peut se lier de manière spécifique à l'ARN transcrit par cette séquence de cADN, d'où la synthèse de la protéine codée par cette séquence de cADN est réduite ou inhibée.

6. Vecteur d'expression contenant la séquence de cADN selon l'une des revendications 1 à 3 ou l'ADN codant le ribozyme selon la revendication 4 ou l'ARN anti-sens selon la revendication 5.

7. Cellule hôte, qui est transformée par le vecteur d'expression selon la revendication 6.

8. Protéine MTR1, qui comporte l'une des séquences d'acide aminé illustrées à la figure 4.

9. Procédé pour obtenir une protéine MTR1 selon la revendication 8, qui comprend la culture de la cellule hôte selon la revendication 7 dans des conditions appropriées et l'obtention de la protéine à partir de la cellule ou du milieu de culture.

10. Anticorps ou fragment de celui-ci, qui se lie de manière spécifique à la protéine MTR1 selon la revendication 8, la protéine MTR1 se composant de l'une des séquences d'acide aminé illustrées à la figure 4.

11. Médicament, qui contient une séquence de cADN selon l'une des revendications 1 à 3, le ribozyme selon la revendication 4, l'ARN anti-sens selon la revendication 5, le vecteur d'expression selon la revendication 6, la protéine MTR1 selon la revendication 8 ou l'anticorps ou le fragment de ce dernier selon la revendication 10.

12. Utilisation de la séquence de cADN selon l'une des revendications 1 à 3, du ribozyme selon la revendication 4, de l'ARN anti-sens selon la revendication 5, du vecteur d'expression selon la revendication 6, de la protéine MTR1 selon la revendication 8 ou de l'anticorps ou du fragment de ce dernier selon la revendication 10 pour produire un médicament pour la prévention ou le traitement de maladies associées à une expression modifiée de la séquence d'ADN codant la MTR1, une activité modifiée de la protéine MTR1 ou une régulation défectueuse de l'entrée de Ca²⁺ dans les cellules.

13. Procédé de diagnostic destiné à démontrer une expression de MTR1 éventuellement perturbée, dans lequel on amène un échantillon en contant avec la séquence de cADN selon l'une des revendications 1 à 3 ou l'anticorps ou le fragment de ce dernier selon la revendication 10 et on détermine, directement ou indirectement, si la concentration, la longueur et/ou la séquence de la protéine MTR1 selon la revendication 8 ou codée par la séquence de cADN selon la revendication 1 ou 2 ou de l'ARN codant cette protéine diffère par rapport à un échantillon témoin.

14. Kit de diagnostic pour mettre en oeuvre le procédé selon la revendication 13, qui contient la séquence de cADN selon l'une des revendications 1 à 3 et/ou l'anticorps ou le fragment de ce dernier selon la revendication 10.
